(19) Europäisches Patentamt
European Patent Office
Office européen des brevets

(11) Veröffentlichungsnummer : **0 034 257**
**B1**

(12) **EUROPÄISCHE PATENTSCHRIFT**

(45) Veröffentlichungstag der Patentschrift : **26.01.83**

(51) Int. Cl.³ : **C 07 C120/00**, C 07 C121/78, C 07 C 97/24

(21) Anmeldenummer : **81100329.2**

(22) Anmeldetag : **17.01.81**

(54) **Verfahren zur Herstellung von 1.4-Diamino-2.3-dicyano-anthrachinon.**

(30) Priorität : **01.02.80 DE 3003656**

(43) Veröffentlichungstag der Anmeldung : **26.08.81 Patentblatt 81/34**

(45) Bekanntmachung des Hinweises auf die Patenterteilung : **26.01.83 Patentblatt 83/04**

(84) Benannte Vertragsstaaten : **CH DE FR GB LI**

(56) Entgegenhaltungen :
**EP A 0 023 645**
**DE A 2 524 748**
**DE C 1 108 704**
**DE C 1 142 174**
**DE C 1 155 786**

**HOUBEN-WEY « Methoden der organischen Chemie » 4. Auflage, Band VII/3c, Teil III 1979, GEORG THIEME VERLAG, Stuttgart, Seiten 239 bis 240.**

(73) Patentinhaber : **BAYER AG**
**Zentralbereich Patente, Marken und Lizenzen**
**D-5090 Leverkusen 1, Bayerwerk (DE)**

(72) Erfinder : **Kröck, Friedrich Wilhelm, Dr.**
**Gerstenkamp 12**
**D-5000 Köln 80 (DE)**
Erfinder : **Neeff, Rütger, Dr.**
**Berta-von-Suttner-Strasse 22**
**D-5090 Leverkusen 1 (DE)**

**EP 0 034 257 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 1.4-Diamino-2,5-dicyano-anthrachinon

Gegenstand der Erfindung ist ein Verfahren zur Herstellung von 1.4-Diamino-2.3-dicyano-anthrachinon

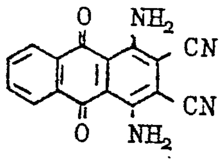

(I)

durch Umsetzung von 1-Amino-4-brom-anthrachinon-2-sulfonsäure oder deren Salzen mit Ammoniak und anschließende Umsetzung der entstandenen 1.4-Diamino-anthrachinon-2-sulfonsäure bzw. deren Salzen mit Cyanidionen abgebenden Verbindungen.

Es ist bereits bekannt, 1.4-Diamino-anthrachinon-2-sulfonsäure oder deren Salze durch Umsetzen von 1-Amino-4-bromanthrachinon-2-sulfonsäure oder deren Salzen mit wäßrigem Ammoniak in Gegenwart von Kupfer oder Kupfersalzen oder mit flüssigem Ammoniak bei 60 bis 100 °C unter einem Druck, bei dem noch flüssiges Ammoniak vorliegt, in Gegenwart von Le A 20 063-Ausland Kupfer, Kupferoxid oder Kupfersalzen herzustellen (DE-PS 1 142 174 und DE-PS 1 155 786). Das erstgenannte Verfahren in wäßrigem Ammoniak ergibt eine sehr unreine 1.4-Diamino-anthrachinon-2-sulfonsäure, da als Nebenprodukt 1-Amino-4-hydroxy-anthrachinon-2-sulfonsäure, die nur sehr schwer abtrennbar ist, in erheblicher Menge entsteht (DE-PS 1 142 174). Die andere Verfahrensvariante weist den Nachteil auf, daß sie mit flüssigem Ammoniak ohne weiteres Lösungsmittel in der Nähe der kritischen Temperatur des Ammoniaks durchgeführt wird. Dabei können vor allem im großtechnischen Maßstab durch Produktabscheidungen an den Gefäßwänden und Überhitzung schwerwiegende Komplikationen auftreten.

Es ist außerdem bekannt, 1.4-Diamino-2.3-dicyano-anthrachinon (I) durch Umsetzung von 1.4-Diamino-anthrachinon-2-sulfonsäure oder deren Salzen mit Cyanidionen abgebenden Verbindungen in wäßrigem Medium herzustellen (DE-PS 556 998 = US-PS 1 938 029). Dieses Verfahren ist später verbessert worden, indem man die Umsetzung in Gegenwart von Luft oder anderen Oxidationsmitteln und/oder Schwermetallkatalysatoren durchführte (vgl. DE-PS 1 108 704 = GB-PS 901 059, DE-PS 1 906 834 = GB-PS 1 212 846 und SU-PS 148 066). Bei der Nacharbeitung der Umsetzung der 1.4-Diamino-anthrachinon-2-sulfonsäure mit Cyanidionen abgebenden Verbindungen in Wasser nach diesen verbesserten Verfahren konnten nie die behaupteten hohen Ausbeuten an reinem 1.4-Diamino-2.3-dicyano-anthrachinon (I) erzielt werden; infolge unvollständiger Austausch- bzw. teilweiser Rückspaltungsreaktionen (vgl. Houben-Weyl, Bd. 7, Teil 3c, Seite 239) wurden stets Reaktionsprodukte erhalten, die größere Mengen an 1.4-Diamino-2-cyano-anthrachinon enthielten, dessen Abtrennung — zumindest in technischem Maßstab — nicht ganz einfach ist.

Bei diesem Stand der Technik kommt eine Zusammenfassung der beiden Reaktionsstufen zu einem « Eintopf-Verfahren » in wäßriger Lösung nicht infrage, weil bereits die getrennten Reaktionsstufen in Wasser nicht ausreichend glatt verlaufen. Ein « Eintopf-Verfahren » wäre aber wünschenswert, nicht zuletzt deshalb, weil die Isolierung der 1.4-Diamino-anthrachinon-2-sulfonsäure wegen ihrer guten Löslichkeit in Wasser und anderen Lösungsmitteln nicht problemlos ist.

Aufgabe der vorliegenden Erfindung war es daher, die Verbindung der Formel I in hohen Ausbeuten und insbesondere hohen Reinheiten aus 1-Amino-4-brom-anthrachinon-2-sulfonsäure oder deren Salzen nach einem möglichst einfachen Verfahren herzustellen.

Erfindungsgemäß wird diese Aufgabe dadurch gelöst, daß man die Umsetzung der 1-Amino-4-brom-anthrachinon-2-sulfonsäure oder deren Salzen mit Ammoniak in Formamid oder N-Methylformamid in Gegenwart von Kupfer, Kupferoxiden und/oder Kupfersalzen und gegebenenfalls in Gegenwart von säurebindenden Mitteln unter Druck durchführt und die Reaktionsmischung nach Abtrennen des überschüssigen Ammoniaks mit Cyanidionen abgebenden Verbindungen, vorzugsweise in Gegenwart eines Oxidationsmittels und gegebenenfalls in Anwesenheit von säurebindenden Mitteln behandelt.

Es ist als außerordentlich überraschend zu bezeichnen, daß die Reaktion der 1-Amino-4-brom-anthrachinon-2-sulfonsäure mit Ammoniak in den erfindungsgemäß zu verwendenden Lösungsmitteln, insbesondere Formamid, so einheitlich und glatt zu 1.4-Diamino-anthrachinon-2-sulfonsäure verläuft, ohne daß das Lösungsmittel mitreagiert und es ist ebenso überraschend, daß die weitere Umsetzung mit Cyanidionen abgebenden Verbindungen in diesen Lösungsmitteln so glatt durchführbar ist, da in anderen technisch gebräuchlichen Lösungsmitteln, wie z.B. Wasser, Dimethylformamid, N-Methylpyrrolidon, Dimethylsulfoxid, Sulfolan, Pyridin, Ethylenglykol, Diethylenglykol, Dimethylacetamid, Tetramethylharnstoff usw. die Ausbeuten unter vergleichbaren Reaktionsbedingungen wesentlich geringer sind.

Die Menge der erfindungsgemäß einzusetzenden Lösungsmittel kann innerhalb eines größeren Bereichs schwanken und hängt im wesentlichen von der Löslichkeit der Reaktionspartner ab. Auf jeden Fall soll sich ein gut rührbares System bilden, in dem die Ausgangsmaterialien vorzugsweise vollständig gelöst sind. Im allgemeinen benötigt man dafür 1 bis 5 kg Lösungsmittel pro Mol 1-Amino-4-brom-anthrachinon-2-sulfonsäure.

1-Amino-4-brom-anthrachinon-2-sulfonsäure und

ihre Salze, insbesondere das Natrium-Salz sind großtechnische Produkte.

Geeignete Kupferkatalysatoren bei der Umsetzung mit Ammoniak sind außer feinverteiltem metallischem Kupfer (sog. Kupferbronze) Cu(I)-oxid, Cu(II)-oxid und Kupfersalze, wie zum Beispiel Kupfercarbonat, basisches Kupfercarbonat, Kupferacetat, basisches Kupferacetat, Kupferformiat, Kupfer(I)-chlorid, Kupfer(II)-chlorid, Kupfer(II)-sulfat, Kupfer(I)-bromid, Kupfer(II)-bromid.

Die Ammoniakmenge, die man pro Mol umzusetzender 1-Amino-4-brom-anthrachinon-2-sulfonsäure benötigt, kann zwischen 100 und 1 000 g schwanken, bevorzugt wird eine Menge von 200 bis 500 g.

Als geeignete säurebindende Mittel können gegebenenfalls insbesondere Natriumcarbonat, Natriumhydrogencarbonat, Kaliumcarbonat, Natriumacetat oder Kaliumacetat hinzugefügt werden, wobei man vorzugsweise die der abzuspaltenden HBr-Menge äquivalente Menge des säurebindenden Mittels zusetzt.

Die Reaktion wird bei 50 bis 100 °C unter dem sich dabei bildenden Druck von maximal 30 bar durchgeführt. Die Reaktionsdauer liegt bei ca. 1 bis 10 Stunden. Danach wird überschüssiges Ammoniak abdestilliert und gegebenenfalls durch Anlegen eines leichten Vakuums weitgehend entfernt. Die gebildete 1.4-Diamino-anthrachinon-2-sulfonsäure wird in Lösung weiterverarbeitet. Das Reaktionsgemisch wird mit Hilfe der Dünnschichtchromatografie auf Vollständigkeit der Umsetzung überprüft.

Für die weitere Umsetzung geeignete Oxidationsmittel sind sowohl anorganische Oxidationsmittel, wie Luft, Sauerstoff, Nitrite, Nitrate, Natriumchlorit, Kaliumbromat, Ammoniumpersulfat, Wasserstoffperoxid und seine Additionsverbindungen, wie Natriumpercarbonat, Natriumperborat und Natriumperpyrophosphat, als auch organische Oxidationsmittel wie zum Beispiel die Additionsverbindung von Harnstoff und Wasserstoffperoxid, Nitrobenzol, Nitromethan m-Nitrobenzoesäure und deren Salze und m-Nitrobenzolsulfonsäure und deren Salze. Bevorzugt verwendet werden Nitromethan, Nitrobenzol, m-Nitro-benzolsulfonsäure und ihre Salze, sowie Sauerstoff, vorteilhaft in Form von Luftsauerstoff, gegebenenfalls in Gegenwart von katalytisch wirkenden Verbindungen, wie Ammoniummolybdat, Ammoniumvanadat oder Kupferverbindungen, z.B. Kupferacetat.

Auf ein Mol der umzusetzenden 1.4-Diamino-anthrachinon-2-sulfonsäure verwendet man zweckmäßigerweise zwischen 0,2 und 2 Mol Nitrobenzol oder m-Nitrobenzol-sulfonsäure, ggf. in Form eines ihrer Salze. Die Menge der anderen angegebenen Oxidationsmittel wird entsprechend ihren Redoxäquivalenten bemessen. Dabei ist ein Unterschuß an Oxidationsmittel wegen der Bildung von Nebenprodukten ebenso zu vermeiden, wie ein größerer Überschuß wegen seiner Wechselwirkung mit den Cyanidionen (vgl. GB-PS 901 059). Im Gegensatz zu den Angaben in diesem Patent, die sich auf wäßrige Reaktionsmedien beziehen, ist es hier bei Verwendung von Formamid oder N-Methyl-formamid allerdings nicht nötig, das Oxidationsmittel während der Reaktion einzudosieren, sondern es kann von Anfang an in der durch Versuche zu ermittelnden optimalen Menge zugegeben werden.

Geeignete säurebindende Mittel sind besonders die bereits zuvor genannten. Ihre Menge ist hauptsächlich davon abhängig, in welcher Form man die Cyanidionen abgebende Verbindung einsetzt, sowie von der bereits in der ersten Stufe zugesetzten Menge. Verwendet man wasserfreie Blausäure, so ist es zweckmäßig, mindestens die äquivalente Menge eines säurebindenden Mittels zuzusetzen. Ein Überschuß an säurebindendem Mittel beeinflußt die Reaktion nicht negativ.

Geeignete Cyanidionen abgebende Verbindungen sind insbesondere Alkalimetall- und Erdalkalimetallcyanide, wie Natrium-, Kalium- oder Magnesiumcyanid, sowie weiterhin Ammoniumcyanid, Zinkcyanid, Kupfer(I)-cyanid, komplexe Kupfer- und Zinkcyanide, Blausäure, Cyanhydrine von Aldehyden und Ketonen. Bevorzugt sind Natriumcyanid und Kaliumcyanid. Die Menge der Cyanidionen abgebenden Verbindung ist so zu bemessen, daß auf 1 Mol der 1.4-Diamino-anthrachinon-2-sulfonsäure bzw. der eingesetzten 1-Amino-4-brom-anthrachinon-2-sulfonsäure oder deren Salze mindestens 2 Mol, vorteilhaft 2,5 bis 10 Mol eingesetzt werden.

Die Reaktionstemperaturen können innerhalb eines größeren Bereichs, d.h. zwischen 40 und 200 °C schwanken. Im allgemeinen arbeitet man in dem für dieses Syntheseprinzip üblichen Temperaturbereich, nämlich zwischen 60 und 120 °C.

Die Reaktion ist beendet, wenn das Dünnschichtchromatogramm die restlose Umsetzung des Ausgangsmaterials anzeigt. Das ist im allgemeinen nach 2 bis 20 Stunden der Fall und hängt von den eingesetzten Mengenverhältnissen und der Temperatur ab. Bei optimaler Dosierung und einer Temperatur von 80 °C ist die Reaktion normalerweise nach 4 Stunden beendet. Die Aufarbeitung ist sehr einfach, da das Dinitril(I) im Gegensatz zu den Ausgangsmaterialien in Formamid ausgesprochen schwer löslich ist, während der Reaktion in gut saugbarer Form auskristallisiert und somit durch Filtration leicht isoliert werden kann.

Aus dem Filtrat kann das Lösungsmittel durch einfache Destillation zurückgewonnen werden, wodurch das Verfahren nicht nur kostensparend ist, sondern auch Abwasserprobleme vermeidet.

Die Reaktionsprodukte fallen unmittelbar in hoher Reinheit an (weniger als 3 % 1.4-Diamino-2-cyano-anthrachinon), was für ihre Verwendung als Ausgangsprodukte zur Herstellung von wertvollen Farbstoffen (vgl. US-PS 26 28 963) von großer Bedeutung ist, da größere Gehalte an 1.4-Diamino-2-cyano-anthrachinon den brillanten, grünstichig blauen Farbton nach weniger erwünschten rotstichigen Nuancen verschieben.

Ein weiterer Vorteil des neuen Herstellungsverfahrens ist darin zu sehen, daß die Verwendung der erfindungsgemäßen Lösungsmittel eine derartige Herabsetzung der Lösungsmittelmenge gestattet, daß die Raum-Zeit-Ausbeute gegenüber den bekannten Verfahren mit Wasser als Lösungsmittel auf etwa den zehnfachen Wert gesteigert werden kann.

Beispiel 1

336,2 g 1-Amino-4-brom-anthrachinon-2-sulfonsäure, Na-salz (85,2 %ig auf freie Säure berechnet), 15 g trockenes Natriumcarbonat und 5 g wasserfreies Kupfersulfat werden in 1,5 l Formamid, das in einem 3 l-Rührautoklaven vorgelegt wird, eingetragen. Der Autoklav wird geschlossen und es werden 350 ccm flüssiges Ammoniak (gemessen unter Druck bei 20 °C ; entspr. 213,5 g) eingepreßt. Das Gemisch wird unter Rühren 8 Stdn. auf 80 °C erhitzt. Druck : ca. 6,2 bar. (Der Druck sinkt im Verlauf der Reaktion etwas ab). Darauf läßt man abkühlen, entspannt den Autoklaven und entfernt überschüssiges Ammoniak durch halbstündiges Evakuieren bei 40 °C. Durch ein Dünnschichtchromatogramm überzeugt man sich, daß die 1-Amino-4-brom-anthrachinon-2-sulfonsäure vollständig umgesetzt ist.

Die so erhaltene Lösung der 1.4-Diamino-anthrachinon-2-sulfonsäure versetzt man mit 110,3 g Natriumcyanid, 45 g Nitrobenzol, 15 g trockenem Natriumcarbonat und 300 ccm Formamid und erhitzt das Gemisch unter Rühren im offenen Gefäß so lange auf 80 °C, bis das Zwischenprodukt restlos umgesetzt ist. Dies ist nach spätestens 4 Stdn. der Fall.

Das auskristallisierte Reaktionsprodukt wird noch heiß abgesaugt, mit 250 ccm Formamid gewaschen und dann mit 500 ccm Methanol und so viel heißem Wasser nachgewaschen, daß der Ablauf praktisch farblos ist. Dazu benötigt man etwa 2-3 l Wasser. Nach dem Trocknen bei 60 °C erhält man 195 g eines dunkelblauen, kristallinen Produkts, das laut Analyse 88,8 % 1.4-Diamino-2,3-dicyano-anthrachinon und 1,3 % 1.4-Diamino-2-cyano-anthrachinon enthält. Das entspricht einer Reinausbeute von 80,1 % Dicyano-Verbindung.

Das Formamid kann aus der Mutterlauge durch Destillation zurückgewonnen und anschließend wieder für die Umsetzung eingesetzt werden. Statt Natriumcarbonat können z.B. bei der Reaktion mit gleichem Erfolg auch entsprechende Mengen Natriumhydrogencarbonat, Kaliumcarbonat, Natriumacetat oder Kaliumacetat eingesetzt werden. Ebenso kann das bei der Umsetzung von 1-Amino-4-brom-anthrachinon-2-sulfonsäure mit Ammoniak als Katalysator verwendete wasserfreie Kupfersulfat auch durch die gleiche Menge Kupferacetat oder Cu(I)-chlorid ersetzt werden, wobei ebenfalls glatte Umsetzungen erzielt werden können. Derselbe Effekt wird auch durch Kupfercarbonat, basisches Kupfercarbonat, basisches Kupferacetat, Kupferformiat, Kupfer(II)-chlorid, Kupfer(I)-bromid, Kupfer(II)-bromid, Kupfer(I)-oxid, Kupfer(II)-oxid oder metallisches, fein verteiltes Kupfer (sogenannte Kupferbronze) erzielt.

Beispiel 2

336,2 g 1-Amino-4-brom-anthrachinon-2-sulfonsäure, Na-salz (85,2 %ig auf freie Säure berechnet) und 5 g Kupfer(I)-chlorid werden in 1,5 l Formamid, das in einem 3 l-Rührautoklaven vorgelegt wird, eingetragen. Der Autoklav wird geschlossen und es werden 350 ccm flüssiges Ammoniak (gemessen unter Druck bei 20 °C ; entspr. 213,5 g) eingepreßt. Das Gemisch wird unter Rühren 8 Stdn. auf 80 °C erhitzt. Druck : ca. 6,4 bar, im Verlauf der Reaktion um ca. 1 bar absinkend. Darauf läßt man abkühlen, entspannt den Autoklaven und entfernt überschüssiges Ammoniak durch halbstündiges Evakuieren bei 40 °C. Durch ein Dünnschichtchromatogramm überzeugt man sich, daß die Umsetzung vollständig ist.

Die erhaltene Reaktionslösung versetzt man mit 110,3 g Natriumcyanid, 15 g trockenem Natriumhydrogencarbonat, 109,8 g trockenem Natrium-m-nitro-benzolsulfonat und 300 ccm Formamid und erhitzt das Gemisch unter Rühren im offenen Gefäß so lange auf 80 °C, bis nach etwa 4 Stdn. das Zwischenprodukt vollständig umgesetzt ist.

Das auskristallisierte Reaktionsprodukt wird wie in Beispiel 1 beschrieben abgesaugt, gewaschen und getrocknet. Man erhält 191 g eines dunkelblauen, kristallinen Produkts, das laut Analyse 88,1 % 1.4-Diamino-2,3-dicyano-anthrachinon und 0,9 % 1.4-Diamino-2-cyano-anthrachinon enthält. Die Reinausbeute an Dicyanoverbindung beträgt 77,9 %.

Statt Natriumcyanid können auch die entsprechenden Mengen der folgenden Cyanidionen abgebenden Verbindungen mit demselben Erfolg eingesetzt werden : Kaliumcyanid, Magnesiumcyanid, Zinkcyanid oder Ammoniumcyanid.

Beispiel 3

Wiederholt man das Beispiel 2 und ersetzt das Natriumcyanid in der zweiten Stufe durch 192 g Aceton-cyanhydrin und gibt außerdem noch 120 g trockenes Natriumcarbonat hinzu, so erhält man 185 g eines dunkelblauen, kristallinen Produkts, das laut Analyse 88,0 % 1.4-Diamino-2,3-dicyano-anthrachinon und 1,3 % 1.4-Diamino-2-cyano-anthrachinon enthält. Die Reinausbeute an Dicyanoverbindung beträgt 75,3 %.

Acetoncyanhydrin kann mit ähnlichem Erfolg auch durch die entsprechende Menge wasserfreier Blausäure ersetzt werden. Ebenso können auch Kupfer(I)-cyanid und Zinkcyanid sowie die komplexen Kupfer- und Zinkcyanide, wie zum Beispiel Kalium-tetracyanocuprat(II), als Cyanidionen abgebende Verbindungen eingesetzt werden.

Beispiel 4

112,1 g 1-Amino-4-brom-anthrachinon-2-sulfonsäure, Na-salz (85,2 %ig auf freie Säure bezogen) und 2 g Kupferacetat werden in 500 ccm Formamid, das in einem 1.1-Rührautoklaven vorgelegt wird, eingetragen. Der Autoklav wird geschlossen und es werden 100 ccm flüssiges Ammoniak (gemessen unter Druck bei 20 °C ; entsprechend 61,0 g) eingepreßt. Das Gemisch wird unter Rühren 8 Stdn. auf 80 °C erhitzt. Druck : 5,3 bar, im Verlauf der Reaktion etwas absinkend. Darauf läßt man abkühlen, entspannt und entfernt überschüssiges Ammoniak durch halbstündiges Evakuieren bei 40 °C. Durch ein Dünnschichtchromatogramm überzeugt man sich von der Vollständigkeit der Umsetzung. Die erhaltene Reaktionslösung versetzt man mit 7,6 g Nitromethan, 36,8 g Natriumcyanid, 10,6 g Natriumcarbonat und 100 ccm Formamid und erhitzt das Gemisch unter Rühren im offenen Gefäß so lange auf 80 °C, bis nach ca. 8 Stdn. das Zwischenprodukt vollständig umgesetzt ist.

Das auskristallisierte Reaktionsprodukt wird wie in Beispiel 1 beschrieben abgesaugt, gewaschen und getrocknet. Man erhält 60,5 g eines dunkelblauen, kristallinen Produkts, das laut Analyse 87,3 % 1.4-Diamino-2,3-dicyano-anthrachinon und 1,1 % 1.4-Diamino-2-cyano-anthrachinon enthält. Die Reinausbeute an Dicyanoverbindung ergibt sich zu 73,3 %.

Statt Nitromethan können mit ähnlichem Erfolg auch entsprechende Mengen der folgenden Oxidationsmittel eingesetzt werden : Nitrobenzol, 3-nitro-benzolcarbonsaures Natrium, Natriumnitrit, Natriumnitrat. Ähnliche Ergebnisse werden auch erhalten, wenn man als Oxidationsmittel einsetzt : Natriumchlorit, Kaliumbromat, Ammoniumpersulfat, Kaliumpersulfat, Wasserstoffperoxid, Natriumpercarbonat, Natriumperborat, Natriumperpyrophosphat, Peressigsäure oder die Additionsverbindung aus Harnstoff und Wasserstoffperoxid.

Beispiel 5

87,3 g 1-Amino-4-brom-anthrachinon-2-sulfonsäure, Na-salz (87,5 %ig auf freie Säure bezogen) und 1,4 g wasserfreies Kupfersulfat werden in 400 ccm Formamid, das in einem 1.1-Rührautoklaven vorgelegt wird, eingetragen. Der Autoklav wird geschlossen und es werden 80 ccm flüssiges Ammoniak (gemessen unter Druck bei 20 °C ; entspr. 49 g) eingepreßt. Das Gemisch wird unter Rühren 8 Stdn. auf 80 °C erhitzt. Druck : 4,5 bar, im Verlauf der Reaktion etwas absinkend. Darauf läßt man abkühlen, entspannt den Autoklaven und überzeugt sich durch ein Dünnschichtchromatogramm, daß die Umsetzung vollständig ist.

Die erhaltene Reaktionslösung versetzt man mit 29,4 g Natriumcyanid, 16,4 g trockenem Natriumacetat, 1,36 g Ammoniumvanadat und 80 ccm Formamid und erhitzt unter Durchleiten von 12,8 l Luft/Stunde so lange auf 80 °C, bis das Zwischenprodukt (1.4-Diaminoanthrachinon-2-sulfonsäure) nach ca. 2,5 Stdn. völlig umgesetzt ist.

Das auskristallisierte Reaktionsprodukt wird wie in Beispiel 1 beschrieben abgesaugt, gewaschen und getrocknet. Man erhält 48 g eines dunkelblauen, kristallinen Produkts, das laut Analyse 87,6 % 1.4-Diamino-2,3-dicyano-anthrachinon und 0,9 % 1.4-Diamino-2-cyano-anthrachinon enthält. Das entspricht einer Reinausbeute von 73 % Dicyanoverbindung.

Mit dem gleichen Erfolg kann man statt Ammoniumvanadat 1,4 g Ammoniummolybdat oder 1,0 g Kupferacetat als Katalysator einsetzen.

Beispiel 6

109,1 g 1-Amino-4-brom-anthrachinon-2-sulfonsäure, Na-salz (87,5 %ig auf freie Säure berechnet), 5 g trockenes Natriumcarbonat und 2 g wasserfreies Kupfersulfat werden in 500 ccm N-Methyl-formamid, das in einem 1.1-Rührautoklaven vorgelegt wird, eingetragen. Der Autoklav wird geschlossen, evakuiert und es werden 120 ccm flüssiges Ammoniak (gemessen unter Druck bei 20 °C ; entsprechend 73,2 g) eingepreßt. Das Gemisch wird unter Rühren 8 Stdn. auf 80-85 °C erhitzt. Druck : 6,2 bar, im Verlauf der Reaktion etwas absinkend. Darauf läßt man abkühlen, entspannt und entfernt überschüssiges Ammoniak durch halbstündiges Evakuieren bei 40 °C. Durch ein Dünnschichtchromatogramm überzeugt man sich von der Vollständigkeit der Reaktion.

Die erhaltene Reaktionslösung versetzt man mit 48,8 g Kaliumcyanid, 20 g Nitrobenzol, 20 g Natriumacetat und 100 ccm N-Methyl-formamid und erhitzt das Gemisch so lange unter Rühren auf 80-85 °C, bis nach ca. 8 Stdn. das Zwischenprodukt lt. DC restlos umgesetzt ist. Das Reaktionsgemisch wird wie in Beispiel 1 beschrieben aufgearbeitet. Nach dem Trocknen erhält man 66 g eines dunkelblauen, kristallinen Produkts, das laut Analyse 85,9 % 1.4-Diamino-2,3-dicyanoanthrachinon und kein 1.4-Diamino-2-cyanoanthrachinon enthält. Das entspricht einer Reinausbeute von 78,7 %.

## Ansprüche

1. Verfahren zur Herstellung von 1.4-Diamino-2,3-dicyano-anthrachinon durch Umsetzung von 1-Amino-4-brom-anthrachinon-2-sulfonsäure oder deren Salzen mit Ammoniak und anschließende Umsetzung mit Cyanidionen abgebenden Verbindungen, dadurch gekennzeichnet, daß man als Lösungsmittel Formamid oder N-Methyl-formamid verwendet und die Reaktion als Eintopfverfahren gestaltet.

2. Verfahren gemäß Anspruch 1, dadurch gekennzeichnet, daß man als Lösungsmittel Formamid verwendet.

3. Verfahren gemäß Anspruch 1 und 2, dadurch gekennzeichnet, daß man die erste Reak-

tionsstufe mit Ammoniak in Gegenwart von Kupfersulfat, Kupferacetat oder Kupfer(I)-chlorid als Katalysatoren und gegebenenfalls in Gegenwart von säurebindenden Mitteln durchführt.

4. Verfahren gemäß Anspruch 1 und 2, dadurch gekennzeichnet, daß man die zweite Reaktionsstufe mit Cyanidionen abgebenden Verbindungen in Gegenwart von Oxidationsmitteln und gegebenenfalls Schwermetallkatalysatoren und säurebindenden Mitteln durchführt.

5. Verfahren gemäß Anspruch 1 und 4, dadurch gekennzeichnet, daß man als Oxidationsmittel Luft, Sauerstoff, Nitrobenzol oder 3-Nitrobenzol-sulfonsäure bzw. deren Salze verwendet.

6. Verfahren gemäß Anspruch 1, 4 und 5, dadurch gekennzeichnet, daß man als Oxidationsmittel Luft oder Sauerstoff in Gegenwart von Schwermetallkatalysatoren, wie Vanadaten, Molybdaten oder Kupfersalzen verwendet.

7. Verfahren gemäß Anspruch 1, 4 und 5, dadurch gekennzeichnet, daß man als Oxidationsmittel Nitrobenzol verwendet.

8. Verfahren gemäß Anspruch 1, dadurch gekennzeichnet, daß man als Cyanidionen abgebende Verbindungen Alkalimetall- oder Erdalkalimetall-cyanide verwendet.

9. Verfahren gemäß Anspruch 1 und 8, dadurch gekennzeichnet, daß man als Cyanidionen abgebende Verbindungen Natriumcyanid oder Kaliumcyanid verwendet.

**Claims**

1. Process for the preparation of 1-4-diamino-2,3-dicyano-anthraquinone by reacting 1-amino-4-bromoanthraquinone-2-sulphonic acid, or its salts, with ammonia and then reacting the resulting product with compounds which yield cyanide ions, characterised in that formamide or N-methyl-formamide is used as the solvent and the reaction is designed as a one-pot process.

2. Process according to Claim 1, characterised in that formamide is used as the solvent.

3. Process according to Claim 1 and 2, characterised in that the first reaction step with ammonia is carried out in the presence of copper sulphate, copper acetate or copper (I) chloride as catalysts and, if appropriate, in the presence of acid-binding agents.

4. Process according to Claim 1 and 2, characterised in that the second reaction step with compounds which yield cyanide ions is carried out in the presence of oxidising agents and, if appropriate, heavy metal catalysts and acid-binding agents.

5. Process according to Claim 1 and 4, characterised in that air, oxygen, nitrobenzene or 3-nitro-benzene-sulphonic acid or its salts is used as the oxidising agent.

6. Process according to Claim 1, 4 and 5, characterised in that air or oxygen is used as the oxidising agent, in the presence of heavy metal catalysts, such as vanadates, molybdates or copper salts.

7. Process according to Claim 1, 4 and 5, characterised in that nitrobenzene is used as the oxidising agent.

8. Process according to Claim 1, characterised in that alkali metal or alkaline earth metal cyanides are used as the compounds which yield cyanide ions.

9. Process according to Claim 1 and 8, characterised in that sodium cyanide or potassium cyanide are used as the compounds which yield cyanide ions.

**Revendications**

1. Procédé de production de 1.4-diamino-2.3-dicyano-anthraquinone par réaction d'acide 1-amino-4-bromoanthraquinone-2-sulfonique ou de ses sels avec l'ammoniac et réaction subséquente avec des composés cédant des ions cyanure, caractérisé en ce qu'on utilise comme solvant le formamide ou le N-méthylformamide et on donne à la réaction l'allure d'un procédé en un seul récipient.

2. Procédé suivant la revendication 1, caractérisé en ce qu'on utilise le formamide comme solvant.

3. Procédé suivant les revendications 1 et 2, caractérisé en ce que la première étape réactionnelle est conduite avec de l'ammoniac en présence de sulfate de cuivre, d'acétate de cuivre ou de chlorure de cuivre (I) comme catalyseurs et, le cas échéant, en présence d'accepteurs d'acides.

4. Procédé suivant les revendications 1 et 2, caractérisé en ce que la seconde étape réactionnelle est conduite avec des composés cédant des ions cyanure en présence d'agents oxydants et, le cas échéant, de catalyseurs à base de métaux lourds et d'accepteurs d'acides.

5. Procédé suivant les revendications 1 et 4, caractérisé en ce qu'on utilise comme oxydant l'air, l'oxygène, le nitrobenzène ou l'acide 3-nitrobenzènesulfonique ou ses sels.

6. Procédé suivant les revendications 1, 4 et 5, caractérisé en ce qu'on utilise comme oxydant l'air ou l'oxygène en présence de catalyseurs à base de métaux lourds tels que des vanadates, des molybdates ou des sels de cuivre.

7. Procédé suivant les revendications 1, 4 et 5, caractérisé en ce qu'on utilise le nitrobenzène comme oxydant.

8. Procédé suivant la revendication 1, caractérisé en ce qu'on utilise comme composés cédant des ions cyanure, des cyanures de métaux alcalins ou de métaux alcalino-terreux.

9. Procédé suivant les revendications 1 et 8, caractérisé en ce qu'on utilise comme composés cédant des ions cyanure, le cyanure de sodium ou le cyanure de potassium.